# EUROPEAN PATENT APPLICATION

(11) **EP 2 407 770 A1**
(43) Date of publication of application: **18.01.2012**
(21) Application number: 11174067.6
(22) Date of filing: 14.07.2011
(51) Int. Cl.: G01N 21/47, G01N 21/49, A61B 5/145, A61B 5/1455, A61B 5/1491, A61B 5/103

(54) **Instrument for measuring a biogenic substance using a confocal optical system**

(30) Priority: 15.07.2010 JP 2010160534
(71) Applicant: Yokogawa Electric Corporation, Tokyo 180-8750 (JP)
(72) Inventor: Tezuka, Shin-ichiro, Tokyo, 180-8750 (JP); Hara, Hitoshi, Tokyo, 180-8750 (JP)
(74) Representative: Henkel, Breuer & Partner

(57) **Abstract**

An instrument for measuring a biogenic substance, using the confocal optical system that is simplified and reducing measurement error caused by the difference in physical conditions or temperatures due to weather of the season is realized. The instrument for measuring a biogenic substance, using the confocal optical system comprises the confocal optical system comprised of a laser source (22), a lens system (23) for collimating light from the laser source (22), a half mirror (24) for causing the light collimated by the lens system (23) to be transmitted therethrough, a lens system (25) for converging the light that is transmitted through the half mirror, a lens system (26), a pinhole (27) and a photodetector (28), and a body for housing these elements and provided with a run button and an exit aperture (44a).

## Description

### BACKGROUND

### Technical Field

The present invention relates to an instrument for measuring a biogenic substance, using the confocal optical system intended for simplifying thereof and improving accuracy thereof.

### Related Art

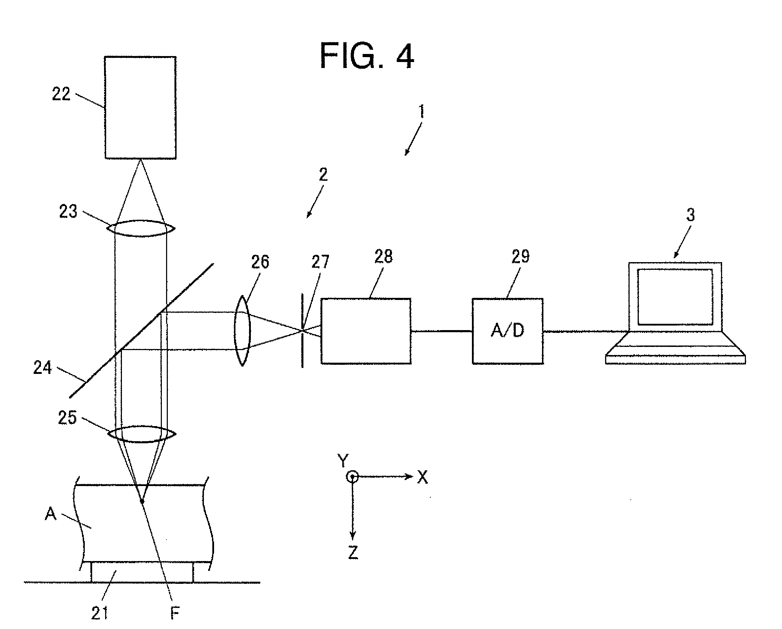

Fig. 4 is a block diagram of a conventional instrument 1 for measuring a biogenic substance, using a confocal optical system. Fig. 4 is briefly explained about hereinafter. The instrument 1 for measuring biogenic substance, using a confocal optical system comprises the confocal optical system 2 for collecting data on a biogenic substance of a biological object A, and a data analysis system 3 for analyzing the data obtained.

The confocal optical system 2 is provided with a placement board 21, on which a biological object A, such as, for example, an arm of a test subject, is placed. Provided above the biological object A is a laser 22 capable of emitting laser beams at two or more wavelengths, and in this case, use is made of a wavelength-variable laser.

A collimator lens 23 for turning a laser beam into parallel rays is disposed in the back stage of the laser 22, and a half mirror 24 having a tilt by approximately 45° against the optical axis is disposed in the back stage of the collimator lens 23, the laser beam being transmitted through the half mirror 24.

An objective lens 25 for converging the parallel rays that that are turned from the laser beam emitted from the laser 22 is disposed in the back stage of the half mirror 24, the laser beam irradiating internal tissues of the biological object A.

Reflected light that is reflected by the internal tissues of the biological object A is refracted by the objective lens 25 to be turned into parallel rays to be subsequently reflected by the half mirror 24, whereupon an optical path thereof is converted by approximately 90°.

Disposed on a side of the half mirror 24 is a lens 26 for receiving the reflected light whose optical path is converted, and converging the same, and the light reflected by the half mirror 24 is converged at the position of a pinhole 27 provided on a side of the lens 26 to pass through the pinhole 27 before being received by a photodetector 28 made up of, for example, a photodiode.

The pinhole 27 can be configured in such a way as to enable a quantity of the reflected light passing therethrough to be adjusted by adoption of, for example, a configuration wherein the pinhole 27 is provided with a diaphragm, a plurality of pinholes having different diameters are provided so as to be switched over among them, and so forth.

Current, and voltage, varying in intensity and magnitude, according to a quantity of the reflected light as received, are outputted as data signals, respectively, from the photodetector 28, and the data signals undergo an A/D conversion by the agency of an A/D converter 29 to be transmitted to the data analysis system 3 of the instrument 1 for measuring a biogenic substance.

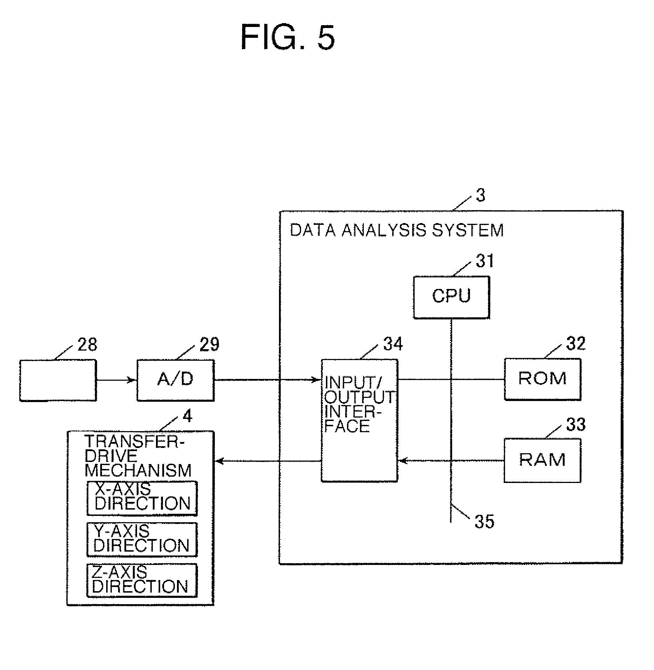

As shown in Fig. 5, the data analysis system 3 is made up of a computer wherein a CPU 31, a ROM 32, a RAM 33, and an input/output interface 34 are connected to a bus 35. The CPU 31 reads various programs stored in the ROM 32, such as a program for data analysis, and so forth, to be expanded as appropriate on the RAM 33, thereby executing various processing.

In data analysis, the CPU 31 executes quantitative determination of the biogenic substance of the biological object A on the basis of a plurality of the data signals inputted from the photodetector 28 via the A/D converter 29, and the input/output interface 34 due to emission of the respective laser beams varying in wavelength, at two or more wavelengths.

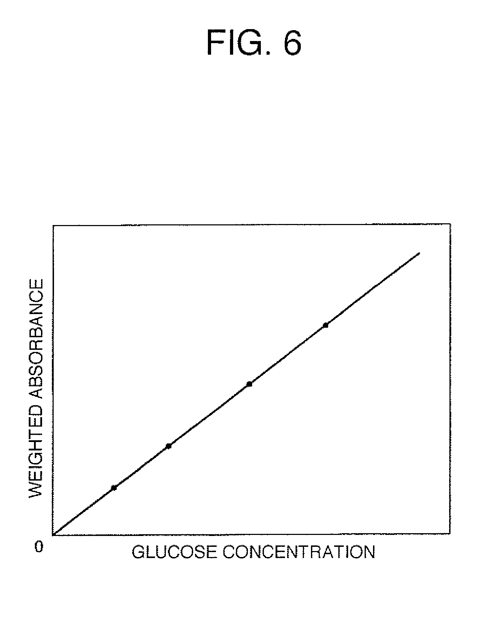

More specifically, in the case of executing quantitative determination of a blood glucose value, that is, the concentration of glucose in blood, since an analytical curve indicating a correlation between the concentration of glucose in blood, and absorbance of a laser beam, as shown in Fig. 6, is stored in the ROM 32, the CPU 31 executes the quantitative determination of the biogenic substance of the biological object A on the basis of the analytical curve.
(Related Art Literature)
(Patent Document 1) JP2008301944A

### SUMMARY OF THE INVENTION

Exemplary embodiments of the present invention address the above disadvantages and other disadvantages not described above. However, the present invention is not required to overcome the disadvantages described above, and thus, an exemplary embodiment of the present invention may not overcome any disadvantages.

It is one of illustrative aspects of the present invention to simplify an instrument for measuring a biogenic substance, using the confocal optical system and to provide the instrument for measuring biogenic substance, using the confocal optical system for reducing measurement error caused by the difference in physical conditions or temperatures of weather of the season.

According to one or more illustrative aspects of the invention, there is provided the instrument for measuring a biogenic substance, using the confocal optical system comprising the confocal optical system comprised of a laser source (hereinafter referred to LD) 22, a lens system 23 for collimating light from the LD 22, a half mirror 24 for causing the light collimated by the lens system 23 to be transmitted therethrough, a lens system 25 for converging the light that is transmitted through the half mirror in the direction of a biological object, a lens system 26 for converging the light that is reflected by the biological object and transmitted through the lens system 25 before reflected by the half mirror 24, a pinhole 27 for causing the light condensed by the lens system 26 to pass therethrough, and a photodetector (hereinafter referred to PD) 28 for receiving the light that is transmitted through the pinhole 27, and a body for housing these elements of the confocal optical system, wherein the body 40 is provided with a run button 41 for driving the LD 22 and an exit aperture 40a for causing the light condensed by the lens system 25 to pass therethrough.

### BRIEF DESCRIPTION OF THE DRAWINGS

Fig. 1(A) is an external perspective view showing an embodiment of a body of an instrument for measuring a biogenic substance, using the confocal optical system, according to the invention, by way of examples, and Fig. 1(B) is a perspective view showing a main part of the confocal optical system;
Fig. 2(A) is an external perspective view showing another embodiment of a body of an instrument for measuring a biogenic substance, using the confocal optical system, according to the invention, by way of examples, and Fig. 2(B) is a perspective view showing a main part of the confocal optical system;
Fig. 3(A) is sectional view of a window member provided in an exit aperture 40a, and Fig. 3(B) is a side view of a temperature control means;
Fig. 4 is a block diagram of a conventional instrument for measuring a biogenic substance, using a confocal optical system;
Fig. 5 is a block diagram of a data analysis system of the conventional instrument for measuring a biogenic substance, using the confocal optical system; and
Fig. 6 is a graph showing an example of an analytical curve indicating a correlation between glucose concentration, and weighted absorbance.

### DEAILED DESCRIPTION OF EXEMPLARY EMBODIMENTS

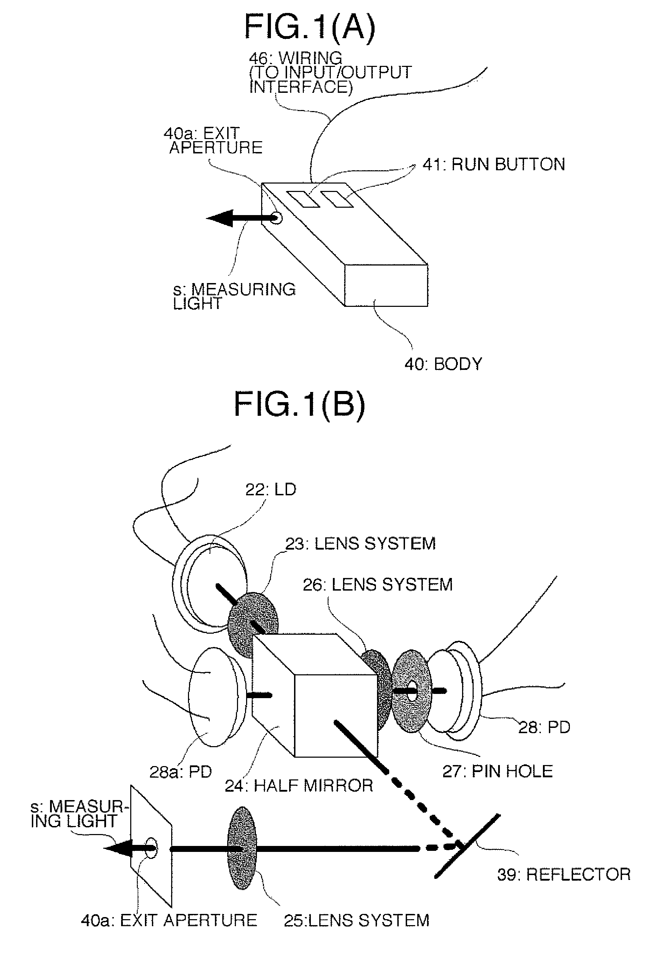

Fig. 1(A) is an external perspective view showing an embodiment of a body of an instrument for measuring a biogenic substance, using a confocal optical system, according to the invention, by way of examples, and Fig. 1(B) is a perspective view showing a main part of the confocal optical system;

The instrument for measuring a biogenic substance, using the confocal optical system has the same configuration and operation as the conventional instrument shown in Fig. 4.

In Figs. 1(A) and 1(B), depicted by 40 is a body formed in a rectangular shape and in which the confocal optical system shown in Fig. 1(B) is housed.

That is, disposed in the body 40 are an LD 22, a collimator lens system 23 disposed in the back stage of the LD 22 for turning a laser beam into parallel rays, a half mirror 24 disposed in the back stage of the collimator lens system 23 and tilted by approximately 45° against the optical axis, a reflector 39 for reflecting the laser beam that is transmitted through the half mirror 24, and a lens system 25 for converging the laser beam that is reflected by the reflector 39.

The converged laser beam is emitted from an exit aperture 40a formed in the body 40 as a measurement light and reflected by an internal tissue of a biological object, not shown. The reflected light falls on the body 40 again and is refracted by the lens system 25 to be turned into parallel rays, and then the parallel rays are reflected by the half mirror 24, whose optical path is converted by approximately 90°.

Disposed on a side of the half mirror 24 is a lens system 26 for converging the reflected light whose optical path is converted, and a plurality of pinholes 27 having different diaphragms and diameters, and a PD 28 for receiving the light passed through the pinholes 27.

Current, and voltage, varying in intensity and magnitude, according to a quantity of the reflected light as received in the same manner as the conventional instrument shown in Fig. 4, are outputted as data signals, respectively, from the PD 28, and the data signals undergo an A/D conversion by the agency of an A/D converter 29 to be transmitted to a data analysis system 3 of the instrument 1 for measuring a biogenic substance. The A/D converter 29 may be provided inside or outside the body 40.

A run button 41 shown in Fig. 1(A) functions as a switch to be connected to the LD22 or the PD 28 that requires a power supplied from an input/output interface 46 as a driving power supply.

With the configuration set forth above, the instrument for measuring a biogenic substance, using the confocal optical system can be downsized because it is housed in the rectangular body 40.

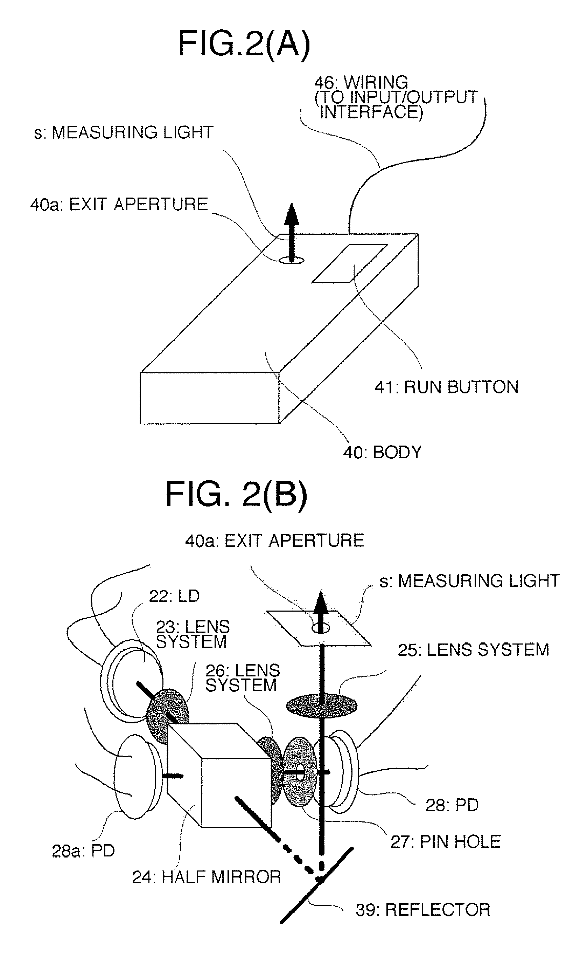

Figs. 2 (A), 2(B) shows another embodiment of the invention wherein a reflecting direction of light that is reflected by the reflector 39 is changed, whereby the light can be emitted from an arbitrary position of the body 40 by changing the mounting direction of the reflector 39.

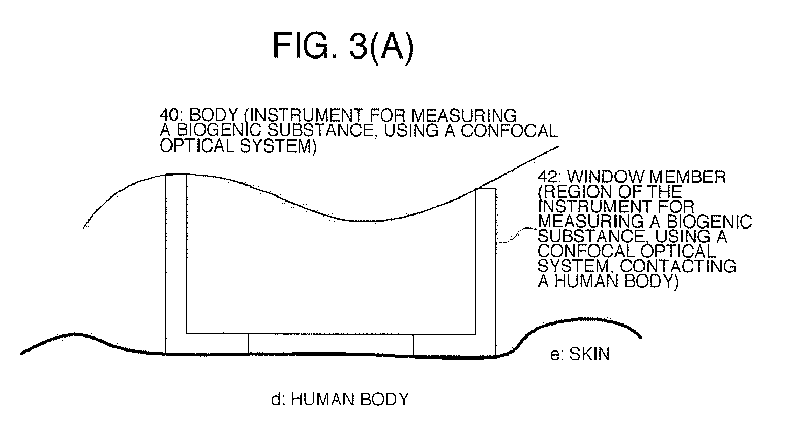

Fig. 3(A) is sectional perspective view of a window member provided in the exit aperture 40a, respectively shown in Fig. 1(A) and Fig. 2(A), and Fig. 3(B) shows a temperature control means as viewed from the direction of an arrow H.

In Fig. 3(A), a window member 42 functions as a region of the instrument for measuring a biogenic substance, using the confocal optical system contacting a human body, and the same figure shows a state where the window member 42 contacts a skin e of a human body d.

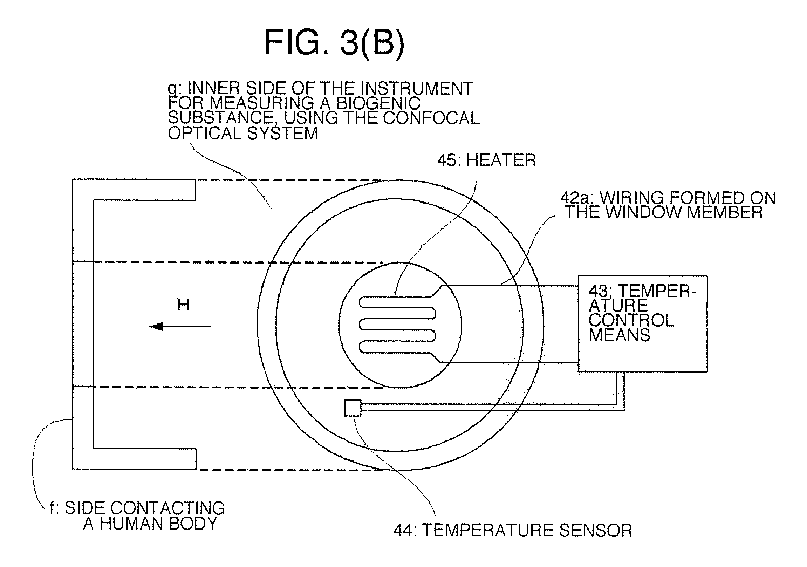

Fig. 3(B) shows a configuration of a temperature control means 43 formed near the center of a cylindrical portion of the window member 42 and it is viewed from the direction of the arrow H.

In the same figure, depicted by 42a is a wiring formed on the window member 42, wherein the wiring 42a is connected to the temperature control means 43 at one end and the other end thereof is connected to a heater 45 made of, for example, Peltier element that is formed in a pectinate shape. Depicted by 44 is a temperature sensor, made up of, for example, a thermistor that is disposed near the center of the window member 42 at a predetermined location not directly heat-affected by the heater.

In the case where the heater 45 is formed of a transparent member, there is no problem in the position of the heater while in the case where the heater 45 is formed of a nontransparent member, it may be formed in a location where light emitted from the lens system 25 (refer to Fig. 1(B) and Fig. 2(B)) is not interrupted by the heater 45.

With the configuration of another embodiment, since a temperature adjustment of the portion to be measured (biological object) is implemented via the window member, it is possible to reduce the error of measurement of a quantity of the biogenic substance caused by a temperature change of the window member per se due to a temperature change in the portion to be measured or seasonal factor.

Further, it is to be pointed out that the foregoing description shows specific and preferred embodiments of the invention for illustrative purposes only. For example, each of the various lenses shown in Fig. 1(B) and 2(B) may be a combination of a plurality of lenses, and the configuration may not be limited to that shown in Fig. 1(B) and 2(B).

As is evident from the configurations of the exemplary embodiments of the instrument for measuring biogenic substance, using the confocal optical system, it is possible to realize the instrument for measuring a biogenic substance, using the confocal optical system that is simplified and reducing measurement error caused by the difference in physical conditions or temperatures due to weather of the season.

While the present invention has been shown and described with reference to certain exemplary embodiments thereof, other implementations are within the scope of the claims. It will be understood by those skilled in the art that various changes in form and details may be made therein without departing from the spirit and scope of the invention as defined by the appended claims.

## Claims

1. An instrument for measuring a biogenic substance, using the confocal optical system comprising the confocal optical system comprised of a laser source (22), a lens system (23) for collimating light from the laser source (22), a half mirror (24) for causing the light collimated by the lens system (23) to be transmitted therethrough, a lens system (25) for converging the light that is transmitted through the half mirror in the direction of a biological object, a lens system (26) for converging the light that is reflected by the biological object and transmitted through the lens system (25) before reflected by the half mirror (24), a pinhole (27) for causing the light condensed by the lens system (26) to pass therethrough, and a photodetector (28) for receiving the light that is transmitted through the pinhole (27), and a body for housing these elements of the confocal optical system, wherein the body (40) is provided with a run button (41) for driving the laser source (22) and an exit aperture (40a) for causing the light condensed by the lens system (25) to pass therethrough.

2. The instrument for measuring a biogenic substance, using the confocal optical system according to claim 1, further comprising a window member (42) provided in the exit aperture (40a) for causing the light to pass therethrough and a temperature control means (43) for controlling a temperature of a surface of the biological object when the window member (42) contacts the biological object.
